# EUROPEAN PATENT SPECIFICATION

(11) **EP 2 642 971 B1**
(45) Date of publication and mention of the grant of the patent: **24.07.2019**
(21) Application number: 11794631.9
(22) Date of filing: 25.11.2011
(51) Int. Cl.: A61K 8/04, A61K 8/43, A61K 8/44, A61Q 9/02, A61Q 19/00

(54) **A SHAVING COMPOSITION FOR PSEUDOFOLLICULITIS BARBAE TREATMENT**
RASIERZUSAMMENSETZUNG ZUR BEHANDLUNG VON PSEUDOFOLLICULITIS BARBAE
COMPOSITION DE RASAGE POUR LE TRAITEMENT DE LA PSEUDOFOLLICULITE DE LA BARBE

(30) Priority: 25.11.2010 DK 201070506
(43) Date of publication of application: 02.10.2013
(73) Proprietor: Shabenaa Holding ApS, 2820 Gentofte (DK)
(72) Inventor: NIELSEN, Nana Rytter, DK-2300 Copenhagen S (DK)
(74) Representative: AWA Denmark A/S
(86) International application number: PCT/DK2011/050449
(87) International publication number: WO 2012/069061

(56) References cited:
- WO-A1-95/13788
- GB-A- 1 209 595
- GB-A- 1 479 708
- US-A- 5 747 021
- US-A1- 2002 141 960

## Description

The present invention relates to a shaving composition comprising an active ingredient for the use in the treatment and/or prevention of pseudofolliculitis barbae and/or folliculitis. The present invention also relates to the use of an active ingredient for the manufacture of a composition for the treatment and/or prevention of the aforementioned conditions.

### Background of the invention

Pseudofolliculitis barbae (PFB) or shaving bumps is a foreign body inflammatory reaction involving papules and pustules. PFB is most common on the male face, but it can also happen on other parts of the body where hair is shaved or plucked, especially areas where hair is curly and the skin is sensitive. A similar condition may also occur after pubic hair is shaved or removed, and this similar condition is termed pseudofolliculitispubis (PFP).

Hair removal such as shaving and epilation of body hair is common. Body hair may be removed from most parts of the body including the head, beard, back, arms, legs, armpits, and in and around the pubic region, the latter is often referred to as bikini waxing. Hair removal in some of these areas such as shaving beard and head is typically limited to males, whereas hair removal in other areas is most commonly performed by females by the use of wax or shaving. Especially, with the decreasing size of women's swimwear, pubic hair which may become visible around the crotch area of a swimsuit is at times removed as such visible pubic hair is widely culturally disapproved of and generally considered to be unaesthetic, undesirable and embarrassing. However, some people also remove pubic hair that is not exposed, for aesthetic or other reasons.

Body hair can be removed in a number of ways, including waxing, shaving, sugaring or using chemical depilatory creams. Hair that is not removed may be trimmed and/or shaved to various lengths. While mainly associated with females, men at times also remove body hair other than the beard and head.

Pseudofolliculitis barbae is often linked to the condition termed folliculitis. Folliculitis is the inflammation of one or more hair follicles. The condition may occur anywhere on the skin. Folliculitis starts when hair follicles are damaged e.g. by shaving. In most cases of folliculitis, the damaged follicles are then infected with the bacterium, typically Staphylococcus.

Two mechanisms are involved in the pathogenesis of pseudofolliculitis barbae: (1) extrafollicular penetration occurs when a curly hair re-enters the skin, and (2) transfollicular penetration occurs when the sharp tip of a growing hair pierces the follicle wall. Tightly curved hair such as pubic hair is predisposed to the condition. The sharp pointed hair from a recent shave briefly surfaces from the skin and re-enters a short distance away. Several methods of close shaving result in a hair cut below the surface. These methods include pulling the skin taut while shaving, shaving against the grain, plucking hairs with tweezers, removing hairs with electrolysis, and using double- or triple-bladed razors. The close shave results in a sharp tip below the skin surface, which is then more likely to pierce the follicular wall, thus causing pseudofolliculitis barbae with transfollicular penetration.

Although usually not regarded as a serious medical problem, pseudofolliculitis barbae can cause cosmetic disfigurement. The papules can lead to multiple papules, scarring, postinflammatory hyperpigmentation, secondary infection, and keloid formation and/or keloid scarring. PFB may also cause pain. The primary lesion is a flesh-colored or erythematous papule with a hair shaft in its center. If the hair shaft is gently lifted up, the free end of the hair comes out of the papule. These inflammatory papules are seen in shaved areas adjacent to the follicular ostia. Pustules and abscess formation can occur from secondary infection. Post-inflammatory hyperpigmentation, scarring, and keloid formation may occur in chronic or improperly treated cases.

Pseudofolliculitis barbae is a recognised problem in both the pharmaceutical and cosmetics industry. Current therapies of PFB include topical treatment with corticosteroids, antibiotics, antibacterials, antiseptics etc.

US 2002/141960 discloses a shaving composition containing a skin-tightening effective amount of an astringent additive, wherein the shaving composition is applied onto human skin prior to shaving. While any suitable astringent compound may be employed such as, for example, witch hazel extract or zinc paraphenolsulfonate, a particularly effective astringent additive is a mixture of butylene glycol and Fomes Officinalis (mushroom extract) commercially available from Laboratories Serobiologiques under the tradename LARICYL® LS 8865.

US 5,308,611 relates to an antiseptic composition comprises a cidal agent and at least one nonionic surfactant selected from the group comprising alkyl phenyl macrogol ethers. Preferably, the cidal agent is chlorhexidine. It has been found that this composition beneficially reduces the number of microrganisms at the application site as measured by Minimum Lethal Concentration (MLC studies. This composition is particularly effective against many aerobic and anaerobic Gram positive and gram negative bacteria including but not limited to those of the genera Corynebacterium, Enterococcus, Staphloycoccus, Streptococcus, Escherichia, and Klebsiella and fungi including but not limited to those of the genus Candida.

GB 1,209,595 discloses a liquid non-lathering shaving preparation consists of an aqueous solution of (a) glycerol or other alcohol obtained by the hydrolysis of an ester by an alkali during a saponification reaction and (b) sodium carboxymethyl cellulose gel or another gel formed from a colloidal solution on standing, the amount of (a) being not greater than 10% v./v. and the amount of (b) being not greater than 5% w./v. The composition may also contain up to 1% w./v. of an antiseptic, e.g. chlorhexidine gluconate or a water-soluble perfume.

WO 01/85112 relates to a shaving cream formulation comprising an anionic surfactant. The formulation may also include an antiseptic. Because these formulations soften the hair, they are soothing, non-irritating, prevent ingrown hairs and provide for greater shaving comfort that conventional shaving creams.

US 4,944,939 relates to a shaving preparation to aid in prevention and treatment of PFB, which include salicylic acid, a glucocorticoid in an amount effective to reduce inflammation; and sulfur. In the shaving preparation the glucocorticoid can be, for example, triamcinolone acetate, hydrocortisone USP, or hydrocortisone acetate USP, and the sulfur can be in precipitated, sublimed or colloidal form.

US 2004180854 relates to oligosaccharide aldonic acids and their topical use. In particular, it relates to such compositions for general care as well as the treatment and prevention of various cosmetic conditions and dermatological disorders, including cosmetic disorders such as pseudofolliculitis barbae. The composition may further comprise antibacterials and/or antiseptics e.g. chlorhexidine.

A shaving solution is described in US 5,387,412 comprising 1,2-propylene glycol in aqueous solution. The shaving solution is described as well suited for people who suffer from mild forms of pseudofolliculitis barbae, and is applied to the area to be shaved prior to shaving.

Another composition comprising propylene glycol for the treatment of dermatological disorders including ingrown hairs i.e. pseudofolliculitis barbae is presented in US 2003165577.

US 5,747,021 describes a topical agent for treating pseudofolliculitis barbae, which in addition to propylene glycol also comprises isopropyl alcohol.

WO 9715282 relates to a composition for the treatment of dermatological disorders associated with changes in normal keratinisation, epidermal formation or pilosebaceous function. The composition comprises an absorbable topical antimicrobial, antibiotic, antibacterial or antifungal agent, and may be used in the treatment of e.g. ingrown hairs, pseudofolliculitis barbae.

WO 02100361 relates to a shaving preparation for the prevention and treatment of pseudofolliculitis barbae and ingrown hairs containing glucocorticoid and an antibacterial agent.

A drawback with several of the above-mentioned treatments relates to the active ingredients. The use of steroid hormones such as glucocorticoids is not preferred, and is often regulated by health authorities i.e. subject to prescription requirements. In some cases long-term use of corticosteroids may have side effects. Also, treatment with antibiotics is generally preserved for more serious infections. Continuous use of a certain antibiotic may in term result in resistant bacteria, thus, rendering the treatment obsolete.

Because of the negative social and sexual consequences for PFB affected individuals, especially related to pseudofolliculitis pubis, and the relative limited numbers of dermal compositions available for prevention and/or topical treatment of ingrown hair, the provision of new compositions for adequate treatment of the dermal condition is required.

The object of the present invention is to provide a topical formulation for application onto the areas, which are subjected to removal of hair; such application resulting in effective reduction of PFB, with the proviso that the topical formulation does not give rise to severe side effects.

### Summary of the invention

A first aspect of the present invention relates to a composition for shaving comprising one or more active ingredient(s) of formula I: 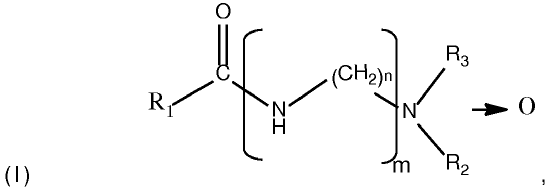 wherein R1 denotes a C1-C25 alkyl group, C3-C7 cycloalkylene group, C1-C25 alkyl C3-C7 cycloalkylene group, C5-C7 arylene group, 5- or 6-membered heteroaryl group, fused heterocyclyl group consisting of two or three rings, or a non-aromatic heterocyclyl group, n denotes an integer from 1 to 6, m denotes 0 or 1, and R2 and R3 represent each independently a C1-C25 alkyl group, and pharmaceutically acceptable salts thereof, for use in the treatment and/or prevention of pseudofolliculitis barbae wherein the antiseptic agent is selected from the group consisting of chlorhexidine, chlorhexidine digluconate, and chlorhexidine gluconate, or mixtures thereof. The compound according to formula I is an amine oxide, which is a chemical compound that contains the functional group R₃N+-O-, an N-O bond with three additional hydrogen and/or side chains attached to N, such side chains may be hydrocarbons and/or carbocyclic ring structures and/or aromatic or non-aromatic heterocyclic ring structures, optionally substituted by an alkyl group. Sometimes such compound is written as R3N→O. According to formula I n may independently be a 1, 2, 3, 4, 5, or 6. R1, R2 and R3 may independently be a C1, C2, C3, C4, C5, C6, C7, C8, C9, C10, C11, C12, C13, C14, C15, C16, C17, C18, C19, C20, C21, C22, C23, C24, or C25 alkyl group, or a C3-C10 carbocyclic ring structure or a C3-C10 heterocyclic ring structure or a bicyclic ring structure.

Surprisingly, the present inventors have found that the above-mentioned active ingredient according to formula I is particular effective in the treatment and/or prevention of PFB. The present inventors found that the active ingredient effectively prevents PFB i.e. the formation of ingrown hair resulting from removal of hair e.g. by shaving of hair. In particular the composition has also proved effective in the prevention of pseudofolliculitis pubis, related to shaving of hair in the pubic region. The active ingredient according to formula I may be any compound within the scope designated hereinbefore. The composition for shaving may comprise one or more active ingredient(s) according to said formula I, and/or any derivatives of the active ingredient(s); including any pharmaceutically acceptable salt thereof.

Without being bound by any specific theory it is believed that the functional group of the active ingredient plays an important role in the prevention of ingrown hair. In particular, the present inventors found that treatment with the active ingredient results in a reduced number of ingrown hair after shaving, and it is believed that the functional group may bind to the terminal hair shaft and/or the skin surface surrounding the terminal hair and thereby facilitates the exit of the growing hair through the skin surface, thus, preventing the hair from curling back or growing sideways into the skin.

Suitable amine oxides include, for example,
(1) almondamidopropylamine oxide (N-[3-(dimethylamino)propyl]almond amides-N-oxide),
(2) babassuamidopropylamine oxide (N-[3-(dimethylamino)propyl]babassu amides-N-oxide),
(3) cocamidopropylamine oxide (N-[3-(dimethylamino)propyl]coco amides-N-oxide),
(4) isostearamidopropylamine oxide (N-[3-(dimethylamino)propyl] isooctadecanamide-N-oxide),
(5) lauramidopropylamine oxide (N-[3-(dimethylamino)propyl] dodecanamide-N-oxide),
(6) myristamidopropylamine oxide (N-[3-(dimethylamino)propyl] tetradecanamide-N-oxide),
(7) oleamidopropylamine oxide (N[3-(dimethylamino)propyl]-9-octadecenamide-N-oxide),
(8) olivamidopropylamine oxide,
(9) palmitamidopropylamine oxide,
(10) sesamidopropylamine oxide,
(11) soyamidopropylamine oxide,
(12) stearamidopropylamine oxide,
(13) tallowamidopropylamine oxide,
(14) undecylenamidopropylamine oxide, and
(15) wheat germamidopropylamine oxide,
as well as mixtures of such amine oxides.

In a preferred embodiment the one or more active ingredient(s) is a compound according to formula I, wherein R1 is a C5-C21 alkyl group, more preferably a C7-C17 alkyl group, even more preferably a C11-C17 alkyl group, R2 is a C1-C10 alkyl group, more preferably a C1-C5 alkyl group, and R3 is a C1-C10 alkyl group, more preferably a C1-C5 alkyl group. R1 may also be selected among 5- or 6-membered heteroaryls such as pyrrolyl, imidazolyl, pyrazolyl, pyridyl, pyridazinyl, pyrimidinyl, oxazolyl, oxadiazolyl, isothiazolyl, thiazolyl, thiadiazolyl, furyl and thienyl; fused heterocyclyl consisting of two rings such as indolyl, isoindolyl, indazolyl, indolizinyl, indolinyl, isoindolinyl, quinolyl, isoquinolyl, cinnolinyl, phthalazinyl, quinazolinyl, naphthyridinyl, quinoxalinyl, purinyl, pteridinyl, benzopyranyl, benzimidazolyl, benzisoxazolyl, benzoxazolyl benbzoxadiazolyl, benzisothiazolyl, benzothiazolyl, benzothiadiazolyl, benzofuryl, isobenzofuryl, benzothienyl, benzotriazolyl, imidazopyridyl, triazoropyridyl, imidazothiazolyl, pyrazinopyridazinyl, quinazolinyl, quinolyl, isoquinolyl, naphthyridinyl, dihydropyridyl, tetrahydroquinolyl and tetxahydrobenzothienyl; fused heterocyclyl consisting of three rings such as carbazolyl, acridinyl, xanthenyl, phenothiazinyl, phenoxathiinyl, phenoxazinyl and dibenzofuryl; and non-aromatic heterocyclyl such as dioxanyl, thiiranyl, oxiranyl, oxathiolanyl, azetidinyl, thianyl, pyrrolidinyl, pyrrolinyl, imidazolidinyl, imidazolinyl, pyrazolidinyl, pyrazolinyl, piperidyl, piperazinyl, morpholinyl, morpholino, thiomorpholinyl, thiomorpholino, dihydropyridyl, tetrahydrofuryl, tetrahydropyranyl, tetrahydrothiazolyl and tetrahydroisothiazolyl.

It has been found that having a compound, wherein R1 is a straight-chain alkyl group of 5 to 21 carbon atoms provides more easy and effective application to the target area. It is believed that the longer alkyl chains may provide a more hydrophobic end of the compound. The opposite end of the compound provides an anionic end due to the amine oxide. This is believed to become a hydrophilic end that bind strongly to the keratin of the hair, thus, making the hydrophobic end of the active ingredient act as the new hair surface. The inventors further found that R2 and R3 may be the same or different. The straight-chain alkyl groups may be shorter compared to R1. It is suggested that shorter alkyl chains facilitate interaction between the amine group and the hair.

In an even more preferred embodiment wherein the compound according to formula I is cocamidopropylamine oxide or lauryldimethyl aminoxide. It has surprisingly been found that the active ingredient being cocamidopropylamine oxide is effective in preventing or substantially reducing PFB caused by removal of hair by shaving. According to formula I both R2 and R3 is a methyl group. Thus, the compound may be enabled to efficiently interact with the area of treatment as the anionic amine oxide is believed to be more exposed. Although the precise chemical effect is not known the inventors found that having n=3 according to formula I provides a very effective active ingredient. Cocamidopropylamine oxide comprises compounds with R1 selected from the group consisting of C5-C17 alkyl groups. It is derived from coconut oil and typically contains about 50% of lauric acid, which corresponds to R1 being a C11 alkyl group. In the present invention the active ingredient may be a mixture of these compounds as the varying length of the R1 alkyl group is believed to provide a better effect on the treatment and/or prevention of PFB.

In a preferred embodiment the composition further comprises an antiseptic agent. The present inventors found that the combination of an active ingredient according to formula I with an antiseptic agent improves the prevention of PFB during and/or subsequent to the removal of hair. Especially, the removal of hair may or may not be accompanied by an infection of the hair follicle (folliculitis), thereby causing "razor bumps". Inward growth of hair which penetrates the epidermis in an arc or pierce the follicular wall may also elicit a foreign body reaction known as a papule. In particular, an infection may occur during removal of hair as the procedure often results in damage to the skin and/or the hair follicle, thus exposing the area to foreign particles and organisms. The human skin constitutes a natural environment for microorganisms such as virus, bacteria and fungi, and damage to the skin and hair follicles disables the barrier so that the infection is facilitated. The inventors found that applying a composition of combined antiseptic agent with the active ingredient according to formula I reduces infection of follicles subsequent to removal of hair e.g. by shaving.

The antiseptic agent is selected from the group consisting of chlorhexidine, chlorhexidine digluconate, and chlorhexidine gluconate, or mixtures thereof. In particular, the present inventors surprisingly found that the addition of an antiseptic agent to the composition enhances the effect of treatment by even further preventing the formation of ingrown hair. The present inventors found that the combination of an active ingredient according to formula I and an antiseptic agent according to the above provides a synergistic effect, thus, reducing the formation of ingrown hair and reducing the occurrence of both folliculitis and pseudofolliculitis barbae more compared to a treatment with either of the two compounds alone. Without being bound by any specific theory it is believed that the application of a surfactant such as a surfactant according to formula I on the skin facilitates the availability of the antiseptic agent. Thus, reducing the number of foreign particles that enter damaged skin and/or follicles and thereby reducing the extent of infection.

In a preferred embodiment the antiseptic agent is chlorhexidine digluconate. Chlorhexidine digluconate is a powerful anti-microbial agent. It is notable for its effectiveness for killing a wide range of bacteria, including both Gram-positive and Gram-negative bacteria. It is also an effective anti-fungal and anti-spore agent, and it is believed to be useful against enveloped viruses. It is believed that the antiseptic agent protects areas with damage to the skin as a result of hair removal by killing microorganisms which would otherwise infect the epidermis, dermis, and/or subcutaneous tissue. The application of chlorhexidine digluconate on skin is well tested and in the correct concentration does not give rise to severe side effects and/or negative effects.

In one embodiment the treatment and/or prevention is specific for pseudofolliculitis barbae which occurs when shaving facial hair and/or hair on the head. Applying the composition while shaving has proved particular useful in preventing or substantially reducing any skin irritation and PFB.

In another embodiment the treatment and/or prevention is specific for pseudofolliculitis barbae related to shaving body hair such as hair on the legs or the back. Use of the composition while shaving these areas has showed effective in preventing or substantially reducing any skin irritation and PFB.

In a particular embodiment the treatment and/or prevention is specific for pseudofolliculitis barbae in the pubic region. It has surprisingly been found that the composition for shaving is particularly useful whilst removing hair in and/or around the pubic region, i.e. preventing or substantially reducing skin irritation, PFB and/or ingrown hair.

The composition according to the invention may comprise any additional additives suitable. In an embodiment of the invention the composition further comprises one or more compounds selected among solvents, surfactants, and excipients, wherein the solvents are selected from the group consisting of isopropyl alcohol, isopropanol, ethanol, n-butanol, n-propanol, methanol, acetic acid, and formic acid, and the surfactants are selected from the group consisting of polypropylene glycol and polyethylene glycol, and the excipients are selected from the group consisting of hydroxypropyl methyl cellulose. The composition provides a pharmaceutically acceptable vehicle for the active ingredient and/or antiseptic agent. It is especially suitable for treatment or prevention of PFB, since the active ingredient and/or antiseptic agent are effectively made available and released to the area of treatment.

The formulation should be suitable for dermal application. In a suitable embodiment the composition is provided in the form of a clear gel, opaque gel, lotion, suspension, ointment, cream, foaming gel, soap, shaving foam or post-foaming gel. In a particular suitable embodiment the composition is provided in the form of a foam. Formulating the composition to such form improves user compliance for dermal application of the active ingredient and/or antiseptic agent as the composition is easily applied to the area of treatment. Especially such forms of the composition are suitable to be applied before, under or after removal of hair e.g. by shaving. In an embodiment of the present invention the composition may be formulated as a shaving preparation such as a soap, a shaving gel foam, a post-foaming gel etc. In a preferred embodiment the composition according to the invention may be applied before and/or during removal of hair. Such application may comprise the composition to be present during the entire removal of hair by e.g. shaving. The inventors surprisingly found that applying of the composition during and/or immediately prior to shaving provide the most efficient prevention and/or treatment of ingrown hair or PFB. Thus, the composition is preferably present during the shaving and may partly or entirely be removed during shaving. More of the composition may be added between shaving passes e.g. when applying wet shaving i.e. shaving with a traditional razor.

The foam, as the preferred form of administering, may be generated in various suitable ways. In one embodiment the precursor liquid of the foam is stored in a pressurised container together with a condensed gas, which, when liberated at normal pressure causes the formation of the foam. In another embodiment, the precursor liquid of the foam is stored in a container at ambient temperature and air is pumped into the precursor thereby causing it to foam. The latter embodiment is preferred due to the absence of potentially dangerous propellants. In a specifically preferred embodiment the air is mechanically pumped into the precursor by physically moving a part of the dispenser. A preferred dispenser of this type includes Rexam's F2 Finger Pump Foamer. The F2 Finger Pump Foamer is part of the Rexam Dispensing System's family of instant, one-touch mechanical foamers. The F2 transforms a precursor liquid into creamy, rich foam with a single push of a button. In an embodiment of the present invention the amount of active ingredient is in the range of 1-50% v/v, more preferably 5-40% v/v, more preferably 7.5-35% v/v, or even more preferably 10-30% v/v.

In another embodiment of the present invention the amount of active ingredient of formula I is in the range of 1-50% v/v, more preferably 5-40% v/v, even more preferably 7.5-35% v/v, and the amount of antiseptic agent is in the range of 1-50% v/v, more preferably 1-40% v/v, more preferably 1-30% v/v, more preferably 1-20% v/v, more preferably 1-15% v/v, even more preferably 1-10% v/v, and again even more preferably 1-5% v/v. In a preferred embodiment the amount of active ingredient of formula I is in the range of 10-30% v/v, and the amount of antiseptic agent is in the range of 1-40% v/v. The present inventors found that a composition comprising the above-mentioned ratios between the one or more active ingredient(s) and the antiseptic agent provides an efficient treatment and/or prevention of PFB.

In another embodiment the composition may be applied in a continuous treatment where the composition is applied before and/or during consecutive shaving of the same region 1, 2, 3, 4, 5, 6, or 7 days a week, or about every 2nd, 3rd, 4th, or 5th day. In particular, the composition has shown effective when it is applied before and/or during consecutive shaving of the same region about every 3rd day.

Another aspect of the present invention relates to the use of one or more active ingredient(s) of formula I: 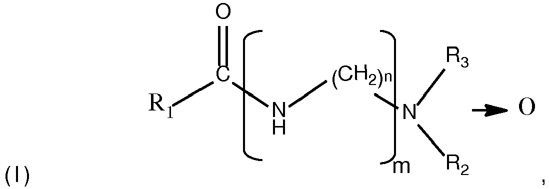 wherein R1 denotes a C1-C25 alkyl group, C3-C7 cycloalkylene group, C1-C25 alkyl C3-C7 cycloalkylene group, C5-C7 arylene group, 5- or 6-membered heteroaryl group, fused heterocyclyl group consisting of two or three rings, or a non-aromatic heterocyclyl group, n denotes an integer from 1 to 6, m denotes 0 or 1, and R2 and R3 represent each independently a C1-C25 alkyl group, and pharmaceutically acceptable salts thereof, for use in the treatment and/or prevention of pseudofolliculitis barbae.

### Detailed description of the invention

Hair is a filamentous biomaterial that grows from follicles found in the dermis. The human body, apart from its glabrous skin, is covered in follicles which produce thick terminal and fine vellus hair. Most common interest in hair is focused on hair growth, hair types and hair care, but hair is also an important biomaterial primarily composed of protein, notably keratin.

Treatment according to the invention may involve all types of hair, which is removed e.g. by shaving. Hairs on different parts of the body resemble each other, but also have characteristics which differentiate them. Thus, head hairs are often long with moderate shaft diameter and diameter variation. Medulla, i.e. the central core of cells that may be present in the hair, is absent to continuous and relatively narrow when compared to the structure of hairs from other body areas. The texture is soft and the hair is pliable. Limb hairs have a fine diameter with little variation. The appearance of the hair is arc-like in shape. Medulla is discontinuous to trace with a granular appearance and the hairs have a soft texture. Facial hairs (beard/mustache) have a very coarse diameter with irregular or triangular cross-sectional shape. Medulla is very broad and continuous and may by doubled. Chest hairs have a shaft diameter. The tip is often darker in color and the hairs are long and fine, arc-like. Medulla may be granular and the hairs have a stiff texture. Axillary or Underarm hairs or hairs in the armpit resemble pubic hairs in general appearance, but is less wiry. Medullary appearance is similar to limb hairs. The diameter is moderate and variable with less buckling than pubic hairs. The tips are long and fine. The hypogastrium or hypogastric region, or pubic region is an area of the human abdomen located below the navel. Pubic hair is hair in the frontal genital area, the crotch, and sometimes at the top of the inside of the legs; these areas form the pubic region. Although fine vellus hair is present in the area in childhood, the term pubic hair is generally restricted to the heavier, longer and coarser hair that develops with puberty as an effect of rising levels of androgens. Pubic hair is therefore part of the androgenic hair. Pubic hair may be characterized by a coarse shaft diameter with wide variations and may often be buckled. The medulla is relatively broad and usually continuous when present. The tip is usually tapered, rounded or abraded. The hair has a stiff and wiry texture.

The terms "hair removal" and "removal of hair" refer to any method of removing and/or reducing hair. Hair can be removed in a number of ways, including waxing, shaving, epilation, sugaring and/or using chemical depilatory creams. Hair that is not removed may be trimmed. These methods include pulling the skin taut while shaving, shaving against the grain, plucking hairs with tweezers, and removing hairs with electrolysis. Removing hair by shaving or trimming may require use of a razor e.g. such as double- or triple-bladed razors, an electrical razor, a hair trimmer or an epilator. An electric razor also referred to as an electric dry shaver has a rotating or oscillating blade. An epilator is an electrical device used to remove hair by mechanically grasping multiple hairs simultaneously and pulling them out. The way in which epilators pull out hair is similar to waxing, although unlike waxing, they do not remove cells from the epithelium of the epidermis. The invention in particular relates to removal of hair by shaving using a shaving tool such as a razor.

When the hair removal is performed by waxing it may not be possible to apply the composition during the procedure. In such case the composition must be applied before and/or after hair removal.

The terms pseudofolliculitis barbae, PFB, barber's itch, folliculitis barbae traumatica, folliculitis pubis, pseudofolliculitis pubis, PFP, razor bumps, scarring pseudofolliculitis, shave bumps may be used interchangeably, and all refer to the medical term for persistent irritation caused by shaving, in particular the medical term for ingrown hair. The term folliculitis is closely related and refers to the inflammation of one or more hair follicles. The condition may occur anywhere on the skin. Folliculitis starts when hair follicles are damaged e.g. by shaving. In most cases of folliculitis, the damaged follicles are then infected with the bacterium or virus.

Pseudofolliculitis barbae (PFB) or shaving bumps is a foreign body inflammatory reaction involving papules and pustules. Curly hair tends to curl into the skin instead of straight out the follicle. PFB can make the skin look itchy and red, and in some cases, it can even look like pimples. These inflamed papules or pustules can form especially if the area becomes infected. Pseudofolliculitis barbae can further be divided into two types of ingrown hairs: transfollicular and extrafollicular.

A papule is a solid raised skin lesion with a distinct border. Papules may have different of shapes and is sometimes associated with other features such as crusts or scales. A pustule is a small collection of pus in the top layer of skin (epidermis) or beneath it in the dermis. Pustules frequently form in sweat glands or hair follicles. Pus is a mixture of inflammatory cells and liquid.

The term "compound", "active compound", "active ingredient", and "one or more active ingredient(s)" may be used interchangeably, and refer to a group of one or more compounds, which may or may not be denoted under a common name, e.g. cocamidopropylamine oxide refers to a composition of compounds derived from coconut oil and comprises a range of different amine oxides with varying length.

The active ingredient, cocamidopropylamine oxide, may also be denoted as *amides, coco, N-[3-(dimethylamino)propyl], N-oxides,* or (N-[3-(dimethylamino)propyl] coco amides-N-oxide). Cocamide or coco amide is a mixture of amides of the fatty acids obtained from coconut oil. As coconut oil may contain about 50% of lauric acid, in formulas only the 12-carbon chains tend to be considered. Therefore, the formula of cocamide can be written as CH₃(CH₂)₁₀CONH₂, though the number of carbon atoms in the chains varies. Coconut oil according to the present invention comprises one or more fatty acids selected from the group consisting of:
(i) lauric acid,
(ii) myristic acid,
(iii) palmitic acid,
(iv) caprylic acid,
(v) capric acid,
(vi) stearic acid,
(vii) caproic acid,
(viii) oleic acid and isomers,
(ix) linoleic acid,
(x) linolenic acid,
(xi) arachidic acid, and
(xii) eicosenoic acid.

Coconut oil contains approximately 92% saturated fatty acids, 6% monounsaturated fatty acids, and 2% polyunsaturated fatty acids, however, these numbers may vary depending on age of the coconut, growing conditions, variety etc. The active ingredient may be a combination of amine oxides according to formula I derived by all or any of the above-mentioned fatty acids.

Surfactants are compounds that lower the surface tension of a liquid, allowing easier spreading, and lowering of the interfacial tension between two liquids, or between a liquid and a solid. Surfactants may act as: detergents, wetting agents, emulsifiers, foaming agents, and dispersants. Any suitable surfactants may be used for carrying out the invention. The surfactants of the present invention may comprise a nontoxic glycol or glycol ether or glycol ester. It may be selected from the group consisting of propylene glycol, butylene glycol or ethylene glycol. Some surfactants that have shown suitable are polyethylene glycol ester and/or ether and polypropylene glycol ester and/or ether surfactants. In a preferred embodiment the surfactant is a polypropylene glycol or polyethylene glycol.

The term "surfactant" and "wetting agent" may be used interchangeably. Examples of wetting agents include, but are not limited to those containing glycerine, ethylene glycol, diethylene glycol, triethylene glycol, polyethylene glycol having a molecular weight of at most 2000, propylene glycol, dipropylene glycol, tripropylene glycol, 1,3-propylene glycol, isopropylene glycol, isobutylene glycol, 1,4-butanediol, 1,3-butanediol, 1,5-pentanediol, 1,6-hexanediol, mesoerythritol, pentaerythritol, ethylene oxide/propylene oxide copolymer and block copolymer, difunctional ethylene oxide/propylene oxide block copolymers containing hydroxyl groups such as primary hydroxyl groups, alkoxylates, alcohol alkoxylates, linear alcohol alkoxylates, monofunctional linear alcohol alkoxylates, pyrrolidone, caprylyl pyrrolidone, and lauryl pyrrolidone.

The composition according to the invention may be formulated in any suitable solvent. Commonly used solvents include isopropyl alcohol, isopropanol, ethanol, n-butanol, n-propanol, methanol, acetic acid, and formic acid. In an embodiment isopropyl alcohol is used as solvent.

The composition according to the present invention may comprise one or more excipients such as methyl hydroxypropyl celluluse also denoted hydroxypropyl methylcellulose. An excipient is generally a pharmacologically inactive substance used as a carrier for the active ingredients of a medication. In many cases, an "active" substance may not be easily administered and absorbed by the human body; in such cases the substance in question may be dissolved into or mixed with an excipient.

The formulation should be suitable for dermal application. Formulations suitable for such application may include liquid or semiliquid preparations such as liniments, lotions, gels, clear gels, oil-in-water or water-in-oil emulsions such as creams, ointments or pastes; or solutions or suspensions such as drops, or foaming gels or post-foaming gels. Especially, the formulation may comprise any form which facilitates the application to and/or availability of the active ingredient and/or antiseptic agent on the skin. The composition of the present invention may be prepared in accordance with methods well known to the person skilled in the art of pharmaceutical and/or cosmetic formulation. Thus, the composition may be prepared by incorporating the ingredients into a well known gel, cream or ointment base. The term "post-foaming gel" means a gel which remains substantially free from foaming for at least a period of 30 seconds after discharge from a pressure tight container under static ambient conditions (about 20°C, at approximately one atmosphere of pressure under conditions substantially free from shearing tension).

By the term applying the composition immediately prior to and/or during removal of hair is meant applying the composition on the skin area of treatment. In particular, the application is similar to the application of a traditional shaving gel and/or cream and/or foaming-gel for traditional shaving of e.g. a beard with a traditional one, two or three-bladed razor.

### Brief disclosure of the drawing

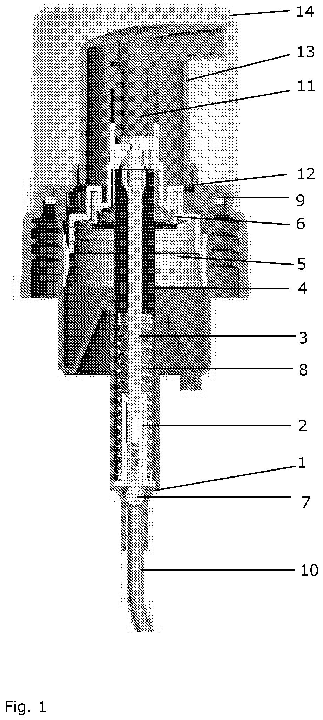

Fig. 1 discloses a dispenser for generating a foam from a precursor.

### Detailed description of the drawing

Fig. 1 discloses a dispenser capable of forming foam from a precursor liquid. The dispenser comprises a diptube 10 for withdrawing a liquid precursor from a container not shown on the drawing. The ball 7, prepared from POM (acetal), is positioned at the upper end of the diptube. A plug 2 is in the vertical direction on the figure positioned on top of the ball. The plug is positioned inside a cylinder 1 and in between the plug and the cylinder a spring 8 is arranged. Above the spring a liquid piston 4 is arranged. The basecap 12 is provided with a thread allowing it to be fastened to a container. Coaxially in the basecap is provided an air piston 5 and gasket 9 for engaging the basecap in the air piston. A valve 6 for activation by pressure is provided at the upper part of the air piston. Above the air piston is provided a netholder 11 comprised in a nozzle housing 13. A overcap 14 is provided for protection of the nozzle.

### Examples

### Composition

A composition according to the present invention may comprise:

**Table 1**

| **Compound** | **Content** |
|---|---|
| Water | > 60% |
| Cocamidopropylamine oxide | 10-30% |
| Chlorhexidin digluconate | 1-5% |
| Isopropyl alcohol | 1-5% |
| Polypropylene glycol | 1-5% |
| Polyethylene glycol | 1-5% |
| Methyl hydroxypropyl cellulose | < 1% |

### Clinical trial

A composition according to the above-mentioned example was tested on 27 test subjects, 4 of which were male. All participants subject to the trails already experienced problems when shaving. 96% of the test subjects participating in the trials already suffered from pseudofolliculitis barbae after shaving and 56% suffered from ingrown hairs after shaving.

Over a period of three weeks each test subject applied the soap according to the above-mentioned composition whilst shaving. The test subjects typically shaved every one to four days. All the test subjects completed the trials.

Table 2 presents an assessment by the test subjects with respect to the effect on pseudofolliculitis barbae and folliculitis of applying the composition before and during shaving. The evaluation of the effect by the test subjects is provided in a graded scale. The scale ranges from 1 to 10, where 1 denotes 'no reduction at all' and 10 denotes complete reduction'. 27 of the test subjects handed in the assessment.

**Table 2**

| **Evaluation** | Percent | **Number of test subjects** |
|---|---|---|
| 10 | 4 % | 1 |
| 9 | 22 % | 6 |
| 8 | 26 % | 7 |
| 7 | 4 % | 1 |
| 6 | 7 % | 2 |
| 5 | 4 % | 1 |
| 4 | 7 % | 2 |
| 3 | 11 % | 3 |
| 2 | 5 % | 1 |
| 1 | 9 % | 2 |
| Do not know | 5 % | 1 |

From the results presented in Table 2 it is evident that 86% of the test subjects to some extent were relieved of pseudofolliculitis barbae and ingrown hairs due to shaving.

### Satisfaction with the product

27 participants of the clinical trials indicated their level of satisfaction with using the composition according to the invention. As presented in Table 3 85% of the test subjects were either satisfied, more than satisfied or very satisfied with the use of the product.

**Table 3**

| | | |
|---|---|---|
| Very satisfied | 10 | 37% |
| More than satisfied | 6 | 22% |
| Satisfied | 7 | 26% |
| Unsatisfied | 3 | 11% |
| Very unsatisfied | 0 | 0% |
| Do not know | 1 | 4% |

## Claims

1. A shaving composition comprising one or more ingredient(s) of formula I: 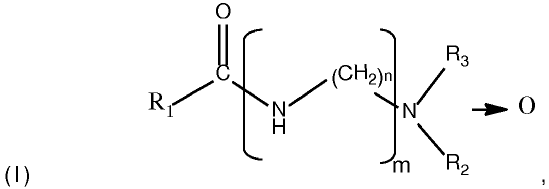 wherein R1 denotes a C1-C25 alkyl group, C3-C7 cycloalkylene group, C1-C25 alkyl C3-C7 cycloalkylene group, C5-C7 arylene group, 5- or 6-membered heteroaryl group, fused heterocyclyl group consisting of two or three rings, or a non-aromatic heterocyclyl group, n denotes an integer from 1 to 6, m denotes 0 or 1, and R2 and R3 represent each independently a C1-C25 alkyl group, and pharmaceutically acceptable salts thereof and further comprises an antiseptic agent, for use in a method for the treatment and/or prevention of pseudofolliculitis barbae, wherein the antiseptic agent is selected from the group consisting of chlorhexidine, chlorhexidine digluconate, and chlorhexidine gluconate, or mixtures thereof.

2. The composition for use according to claim 1, wherein R1 is a C5-C21 alkyl group, more preferably a C7-C17 alkyl group, even more preferably a C11-C17 alkyl group, R2 is a C1-C10 alkyl group, more preferably a C1-C5 alkyl group, and R3 is a C1-C10 alkyl group, more preferably a C1-C5 alkyl group.

3. The composition for use according to any of the preceding claims, wherein formula I is cocamidopropylamine oxide.

4. The composition for use according to claim 3, wherein the antiseptic agent is chlorhexidine digluconate.

5. The composition for use according to any of the preceding claims, wherein the composition further comprises one or more compounds selected among solvents, surfactants, and excipients, wherein the solvents are selected from the group consisting of isopropanol, ethanol, n-butanol, n-propanol, methanol, acetic acid, and formic acid, and the surfactants are selected from the group consisting of polypropylene glycol and polyethylene glycol, and the excipients are selected from the group consisting of hydroxypropyl methyl cellulose.

6. The composition for use according to any of the preceding claims, wherein the composition is provided in the form of a clear gel, opaque gel, lotion, suspension, ointment, cream, foaming gel, soap, shaving foam or post-foaming gel.

7. The composition for use according to any of the claims 1 to 6, wherein the amount of active ingredient of formula I is in the range of 10-30% v/v, and the amount of antiseptic agent is in the range of 1-40% v/v.

## Patentansprüche

1. Rasierzusammensetzung, umfassend einen oder mehrere Bestandteil(e) der Formel I: 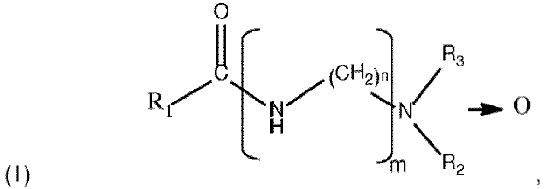 wobei R1 eine C1-C25-Alkylgruppe, C3-C7-Cycloalkylengruppe, C1-C25-Alkyl-C3-C7-cycloalkylengruppe, C5-C7-Arylengruppe, 5- oder 6-gliedrige Heteroarylgruppe, kondensierte Heterocyclylgruppe, die aus zwei oder drei Ringen besteht, oder eine nichtaromatische Heterocyclylgruppe bezeichnet, n eine ganze Zahl von 1 bis 6 bezeichnet, m 0 oder 1 bezeichnet und R2 und R3 jeweils unabhängig eine C1-C25-Alkylgruppe bezeichnen, und pharmazeutisch verträgliche Salze davon, und ferner umfassend ein antiseptisches Mittel, zur Verwendung bei einem Verfahren zur Behandlung und/oder Prävention von Pseudofolliculitis barbae, wobei das antiseptische Mittel ausgewählt ist aus der Gruppe bestehend aus Chlorhexidin, Chlorhexidindigluconat und Chlorhexidingluconat und Gemischen davon.

2. Zusammensetzung zur Verwendung gemäß Anspruch 1, wobei R1 eine C5-C21-Alkylgruppe ist, bevorzugter eine C7-C17-Alkylgruppe, noch bevorzugter eine C11-C17-Alkylgruppe, R2 eine C1-C10-Alkylgruppe ist, bevorzugter eine C1-C5-Alkylgruppe, und R3 eine C1-C10-Alkylgruppe ist, bevorzugter eine C1-C5-Alkylgruppe.

3. Zusammensetzung zur Verwendung gemäß einem der vorstehenden Ansprüche, wobei Formel I Cocamidopropylaminoxid ist.

4. Zusammensetzung zur Verwendung gemäß Anspruch 3, wobei das antiseptische Mittel Chlorhexidindigluconat ist.

5. Zusammensetzung zur Verwendung gemäß einem der vorstehenden Ansprüche, wobei die Zusammensetzung ferner eine oder mehrere Verbindungen ausgewählt aus Lösungsmitteln, grenzflächenaktiven Mitteln und Hilfsstoffen umfasst, wobei die Lösungsmittel ausgewählt sind aus der Gruppe bestehend aus Isopropanol, Ethanol, n-Butanol, n-Propanol, Methanol, Essigsäure und Ameisensäure, und die grenzflächenaktiven Mittel ausgewählt sind aus der Gruppe bestehend aus Polypropylenglycol und Polyethylenglycol, und die Hilfsstoffe ausgewählt sind aus der Gruppe bestehend aus Hydroxypropylmethylcellulose.

6. Zusammensetzung zur Verwendung gemäß einem der vorstehenden Ansprüche, wobei die Zusammensetzung in der Form eines klaren Gels, eines undurchsichtigen Gels, einer Lotion, einer Suspension, einer Salbe, einer Creme, eines schäumenden Gels, einer Seife, eines Rasierschaums oder eines nachschäumenden Gels bereitgestellt wird.

7. Zusammensetzung zur Verwendung gemäß einem der Ansprüche 1 bis 6, wobei die Menge an Wirkstoff der Formel I in dem Bereich von 10-30 % Vol./Vol. liegt und die Menge an antiseptischem Mittel in dem Bereich von 10-40 % Vol./Vol. liegt.

## Revendications

1. Composition de rasage comprenant un ou plusieurs composant(s) de formule I : 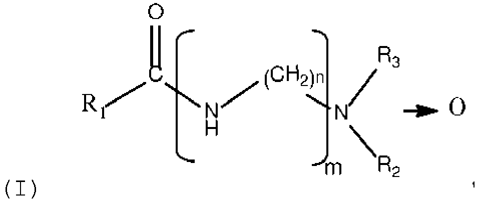 dans laquelle R1 désigne un groupe alkyle en C1-C25, un groupe cycloalkylène en C3-C7, un groupe (alkyle en C1-C25)-(cycloalkylène en C3-C7), un groupe arylène en C5-C7, un groupe hétéroaryle de 5 ou 6 chaînons, un groupe hétérocyclyle condensé constitué de deux ou trois cycles, ou un groupe hétérocyclyle non aromatique, n désigne un entier de 1 à 6, m désigne 0 ou 1, et R2 et R3 représentent chacun indépendamment un groupe alkyle en C1-C25, et des sels pharmaceutiquement acceptables de ceux-ci et comprend en outre un agent antiseptique, pour utilisation dans un procédé pour le traitement et/ou la prévention de la pseudo-folliculite de la barbe, dans laquelle l'agent antiseptique est choisi dans le groupe constitué de la chlorhexidine, le digluconate de et le gluconate de chlorhexidine, ou des mélanges de ceux-ci.

2. Composition pour utilisation selon la revendication 1, dans laquelle R1 est un groupe alkyle en C5-C21, plus préférablement un groupe alkyle en C7-C17, encore plus préférablement un groupe alkyle en C11-C17, R2 est un groupe alkyle en C1-C10, plus préférablement un groupe alkyle en C1-C5, et R3 est un groupe alkyle en C1-C10, plus préférablement un groupe alkyle en C1-C5.

3. Composition pour utilisation selon l'une quelconque des revendications précédentes, dans laquelle la formule I est l'oxyde de cocamidopropylamine.

4. Composition pour utilisation selon la revendication 3, dans laquelle l'agent antiseptique est le digluconate de chlorhexidine.

5. Composition pour utilisation selon l'une quelconque des revendications précédentes, la composition comprenant en outre un ou plusieurs composés choisis parmi des solvants, des tensioactifs et des excipients, dans laquelle les solvants sont choisis dans le groupe constitué des isopropanol, éthanol, n-butanol, n-propanol, méthanol, acide acétique et acide formique, et les tensioactifs sont choisis dans le groupe constitué du polypropylène glycol et du polyéthylène glycol, et les excipients sont choisis dans le groupe constitué de l'hydroxypropylméthylcellulose.

6. Composition pour utilisation selon l'une quelconque des revendications précédentes, la composition étant fournie sous la forme d'un gel limpide, d'un gel opaque, d'une lotion, d'une suspension, d'une pommade, d'une crème, d'un gel moussant, d'un savon, d'une mousse de rasage ou d'un gel post-moussant.

7. Composition pour utilisation selon l'une quelconque des revendications 1 à 6, dans laquelle la quantité de substance active de formule I est dans la plage de 10 à 30 % v/v, et la quantité d'agent antiseptique est dans la plage de 1 à 40 % v/v.
